# EUROPEAN PATENT APPLICATION

(11) **EP 4 445 836 A2**
(43) Date of publication of application: **16.10.2024**
(21) Application number: 24166311.1
(22) Date of filing: 26.03.2024
(51) Int. Cl.: A61B 5/0538, A61B 5/145, A61B 5/1473, A61B 5/1495, A61B 5/00

(54) **SYSTEMS AND METHODS FOR ASSESSING SENSOR SURFACE AREA ENCAPSULATION IN VITRO AND IN VIVO**

(30) Priority: 14.04.2023 US 202363459273 P; 13.03.2024 US 202418603875
(71) Applicant: Medtronic MiniMed, Inc., Northridge, CA 91325 (US)
(72) Inventor: Russell, Raymond M., Northridge, 91325 (US); Jacks, Steven, Northridge, 91325 (US)
(74) Representative: Zimmermann & Partner Patentanwälte mbB

(57) **Abstract**

A glucose sensor includes a working electrode, sensor electronics configured to periodically measure electrode current signal (Isig) values for the working electrode and counter voltage values (Vcntr) for the glucose sensor, one or more processors, and one or more processor-readable media. The one or more processor-readable media stores instructions which, when executed by the one or more processors, cause performance of: generating Electrochemical Impedance Spectroscopy (EIS) parameter values for the working electrode based on an EIS procedure; estimating a reaction surface area of the glucose sensor based on least one of Isig values, Vcntr values, or the EIS parameter values; determining a difference between the estimated reaction surface area and an expected reaction surface area of the glucose sensor; comparing the determined difference to a predetermined threshold value; and adjusting a calibration factor when the determined difference is below the predetermined threshold value.

## Description

### CROSS-REFERENCE TO RELATED APPLICATIONS

This application claims the benefit of and priority to U.S. Provisional Patent Application Serial No. 63/459,273 filed April 14, 2023, the entire disclosure of which is incorporated by reference herein.

### FIELD

The present disclosure relates generally to glucose sensor technology and, more particularly, to glucose sensor systems and methods for detecting glucose sensor performance.

### BACKGROUND

Diabetes mellitus also referred to as diabetes, is a metabolic disease associated with high blood sugar due to insufficient production or use of insulin by the body. Diabetes is widely-spread globally, affecting hundreds of millions of people, and is among the leading causes of death globally. Diabetes has been categorized into three categories or types: type 1, type 2, and gestational diabetes. Type 1 diabetes is associated with the body's failure to produce sufficient levels of insulin for cells to uptake glucose. Type 2 diabetes is associated with insulin resistance, in which cells fail to use insulin properly. The third type of diabetes is commonly referred to as gestational diabetes, which can occur during pregnancy when a pregnant woman develops a high blood glucose level. Gestational diabetes can develop into type 2 diabetes but often resolves after pregnancy.

Continuous glucose monitoring (CGM) is used to continuously monitor glucose levels in a user and alerts the user when the glucose level is outside of the normal range. The glucose levels can be measured based on raw glucose sensor values (e.g., the sensor current). When the glucose level is above a safe range of glucose levels, insulin is injected into the user.

### SUMMARY

The present disclosure relates to systems and methods for detecting glucose sensor performance so that a proper glucose level can be provided.

In accordance with aspects of the present disclosure, a glucose sensor includes a working electrode, sensor electronics configured to periodically measure electrode current signal (Isig) values for the working electrode and counter voltage (Vcntr) values for the glucose sensor, one or more processors, and one or more processor-readable media. The one or more processor-readable media includes instructions stored thereon, which, when executed, cause the performance of generating Electrochemical Impedance Spectroscopy (EIS) parameter values for the working electrode based on an EIS procedure; estimating a reaction surface area of the glucose sensor based on Isig values, Vcntr values, and/or the EIS parameter values; determining a difference between the estimated reaction surface area and an expected reaction surface area of the glucose sensor; comparing the determined difference to a predetermined threshold value; and adjusting a calibration factor when the determined difference is below the predetermined threshold value.

In various aspects, the calibration factor adjustment may be based on the difference between the estimated reaction surface area and the expected reaction surface area.

In various aspects, the one or more processor-readable media further store instructions which, when executed by the one or more processors, may cause the performance of determining if a signal sensitivity is below a predetermined threshold value,

In various aspects, the EIS parameter values may include a real impedance, an imaginary impedance, and/or a magnitude of vector of real and imaginary portions of impedance.

In various aspects, the instructions, when executed by the one or more processors, further cause the glucose sensor to adjust a sample rate for measuring Isig, Vcntr, and/or EIS in response to the signal sensitivity being below the predetermined threshold value.

In various aspects, the EIS parameter may be an imaginary impedance at 0.1 Hz and/or a real impedance at 1KHz.

In various aspects, estimating the reaction surface area of the glucose sensor may include estimating the reaction surface area over time.

In various aspects, the one or more processor-readable media further store instructions which, when executed by the one or more processors, may cause the performance of transmitting a notification providing an indication for a user to move the glucose sensor to a different physical wear location.

In various aspects, the expected reaction surface area of the glucose sensor may be based on an area under a signal curve being proportional to the reaction surface area.

In various aspects, the signal curve may be based on Isig values, Vcntr values, and/or the EIS parameter values over time.

In accordance with aspects of the present disclosure, one or more non-transitory processor readable media storing instructions which, when executed by one or more processors, cause the performance of: periodically measuring electrode current signal (Isig) values for a working electrode and of counter voltage (Vcntr) values for a glucose sensor; generating Electrochemical Impedance Spectroscopy (EIS) parameter values for the working electrode based on an EIS procedure; estimating a reaction surface area of the glucose sensor based on Isig values, Vcntr values, and/or the EIS parameter values; determining a difference between the estimated reaction surface area and an expected reaction surface area of the glucose sensor; comparing the determined difference to a predetermined threshold value; and adjusting a calibration factor when the determined difference is below the predetermined threshold value.

In various aspects, the calibration factor adjustment is based on the difference between the estimated reaction surface area and the expected reaction surface area.

In various aspects, the calibration factor adjustment may be based on the difference between the estimated reaction surface area and the expected reaction surface area.

In various aspects, the EIS parameter values may include a real impedance, an imaginary impedance, and/or a magnitude of vector of real and imaginary portions of impedance.

In various aspects, the EIS parameter may be a real impedance at 1 KHz.

In various aspects, the EIS parameter may be an imaginary impedance at 0.1 Hz.

In various aspects, estimating the reaction surface area of the glucose sensor may include estimating the reaction surface area over time.

In various aspects, the one or more non-transitory processor-readable media store further instructions which, when executed by the one or more processors, may cause the performance of transmitting a notification providing an indication for a user to move the glucose sensor to a different physical wear location.

In various aspects, the expected reaction surface area of the glucose sensor is based on an area under a signal curve being proportional to the reaction surface area. The signal curve may be based on Isig values, Vcntr values, and/or the EIS parameter values over time.

In accordance with aspects of the present disclosure, a system includes one or more processors and one or more processor-readable media storing instructions which, when executed by the one or more processors, cause performance of: periodically measuring electrode current signal (Isig) values for a working electrode and of counter voltage (Vcntr) values for a glucose sensor; generating Electrochemical Impedance Spectroscopy (EIS) parameter values for the working electrode based on an EIS procedure; estimating a reaction surface area of the glucose sensor based on Isig values, Vcntr values, and/or the EIS parameter values; determining a difference between the estimated reaction surface area and an expected reaction surface area of the glucose sensor; comparing the determined difference to a predetermined threshold value; and adjusting a calibration factor when the determined difference is below the predetermined threshold value.

Further disclosed herein is an embodiment of a glucose sensor. The glucose sensor includes a working electrode, sensor electronics configured to periodically measure electrode current signal (Isig) values for the working electrode and counter voltage values (Vcntr) for the glucose sensor, one or more processors, and one or more processor-readable media. The one or more processor-readable media stores instructions which, when executed by the one or more processors, cause performance of: generating Electrochemical Impedance Spectroscopy (EIS) parameter values for the working electrode based on an EIS procedure; estimating a reaction surface area of the glucose sensor based on least one of Isig values, Vcntr values, or the EIS parameter values; determining a difference between the estimated reaction surface area and an expected reaction surface area of the glucose sensor; comparing the determined difference to a predetermined threshold value; and adjusting a calibration factor when the determined difference is below the predetermined threshold value.

The details of one or more aspects of the disclosure are set forth in the accompanying drawings and the description below. Other features, objects, and advantages of the techniques described in this disclosure will be apparent from the description and drawings and from the claims.

### BRIEF DESCRIPTION OF THE DRAWINGS

A detailed description of aspects of the disclosure will be made with reference to the accompanying drawings, wherein like numerals designate corresponding parts in the figures.
FIG. 1 illustrates a perspective view of a subcutaneous sensor set and a block diagram of a glucose sensor electronics device in accordance with aspects of the present disclosure;
FIG. 2 illustrates electrodes for use with the glucose sensor electronics device of FIG. 1, in accordance with aspects of the present disclosure;
FIG. 3 shows a block diagram illustrating an electronic circuit for sensing an output of a sensor, in accordance with aspects of the present disclosure;
FIG. 4 shows a block diagram illustrating a sensor electronics device and a sensor including a plurality of electrodes, in accordance with aspects of the present disclosure;
FIG. 5 shows a block diagram illustrating another embodiment of a sensor electronics device and a sensor including a plurality of electrodes, in accordance with aspects of the present disclosure;
FIG. 6 shows a block diagram illustrating an exemplary approach for detecting glucose sensor performance, in accordance with aspects of the present disclosure;
FIG. 7 is a graph illustrating Isig over time for the electrodes of FIG. 2, in accordance with aspects of the present disclosure;
FIG. 8 is a graph illustrating Isig over time for electrodes with varying amounts of surface area, in accordance with aspects of the present disclosure;
FIG. 9 is a graph illustrating imaginary impedance at 0.1 Hz over time for electrodes with varying amounts of surface area, in accordance with aspects of the present disclosure; and
FIG. 10 is a graph illustrating real impedance at 1 KHz over time for electrodes with varying amounts of surface area, in accordance with aspects of the present disclosure.

### DETAILED DESCRIPTION

In the following description, reference is made to the accompanying drawings which form a part hereof and which illustrate several aspects of the present disclosure. It is understood that other aspects may be utilized, and structural and operational changes may be made without departing from the scope of the present disclosure.

The aspects herein are described below with reference to flowchart illustrations of methods, systems, devices, apparatuses, and programming and computer program products. It will be understood that each block of the flowchart illustrations, and combinations of blocks in the flowchart illustrations, can be implemented by programming instructions, including computer program instructions (as can any menu screens described in the figures). These computer program instructions may be loaded onto a computer or other programmable data processing apparatus (such as a controller, microcontroller, or processor in a sensor electronics device) to produce a machine, such that the instructions which execute on the computer or other programmable data processing apparatus create instructions for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks, and/or menus presented herein. Programming instructions may also be stored in and/or implemented via electronic circuitry, including integrated circuits (ICs) and Application Specific Integrated Circuits (ASICs) used in conjunction with sensor devices, apparatuses, and systems.

As used herein, the term "exemplary" does not necessarily mean "preferred" and may simply refer to an example unless the context clearly indicates otherwise.

When worn on the body, glucose sensors may encounter encapsulation and/or surface area reduction. For example, at least a portion of the glucose sensor may be covered by a biofilm, which may have the effect of reducing the available surface area of the glucose sensor for sensing blood glucose. The disclosed features enable the determination of available sensor area based on the measurement of sensor signals, such as, Isig and/or Electrochemical Impedance Spectroscopy (EIS) parameter values. By determining the reduction in available sensor surface area vs. an expected sensor surface area, the disclosed features of the present disclosure may serve to compensate for the reduction in available sensor surface area of the glucose sensor to ensure accurate and reliable sensor data while potentially extending the life of the glucose sensor.

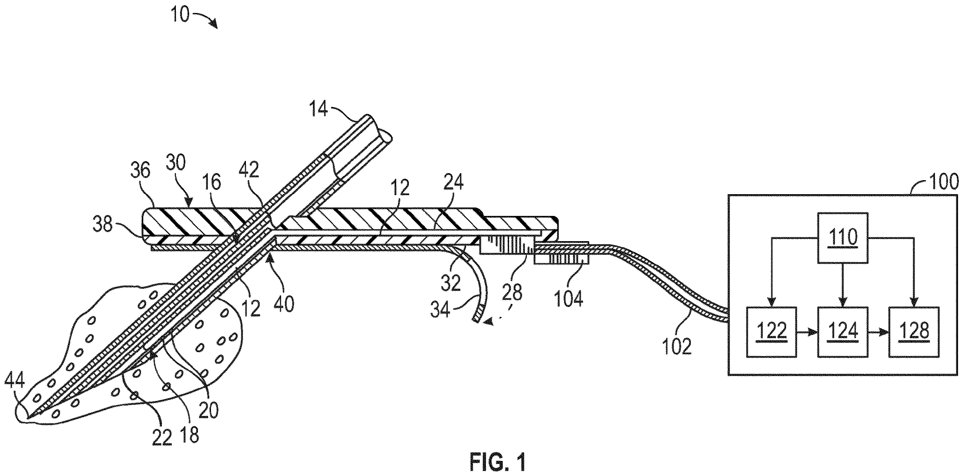

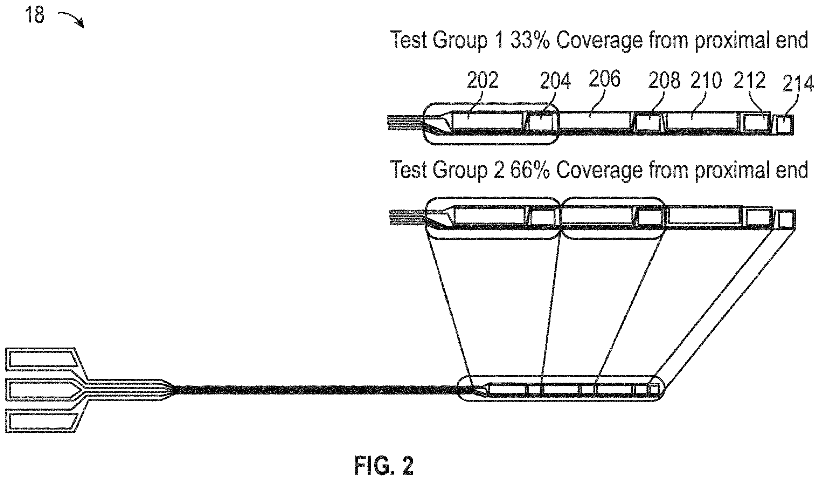

FIG. 1 is a perspective view of a subcutaneous sensor set 10 and a block diagram of a sensor electronics device 100 according to various aspects of the present disclosure. The subcutaneous sensor set 10 is provided for subcutaneous placement of a sensing portion 18 of an electrochemical sensor 12 (FIG. 2) at a selected site in the body of a user. The sensor set 10 includes a hollow, slotted insertion needle 14 and a cannula 16. The needle 14 is used to facilitate quick and easy subcutaneous placement of the cannula 16 at the subcutaneous insertion site. Inside the cannula 16 is a sensing portion 18 of the sensor 12 to expose one or more sensor electrodes 20 to the user's bodily fluids through an opening 22 in the distal portion of the cannula 16. In an aspect of the present disclosure, the one or more sensor electrodes 20 may include a counter electrode, a reference electrode, and one or more working electrodes. After insertion, the insertion needle 14 is withdrawn to leave the cannula 16 with the sensing portion 18 and the one or more sensor electrodes 20 in place at the selected insertion site.

In particular aspects, the sensor set 10 facilitates accurate placement of the electrochemical sensor 12, which is used for monitoring specific blood parameters representative of a user's condition. The electrochemical sensor 12 monitors glucose levels in the body and may be used in conjunction with automated or semi-automated medication infusion pumps of the external or implantable type to control delivery of insulin to a diabetic patient, as described, e.g., in U.S. Patent Nos. 4,562,751; 4,678,408; 4,685,903; and 4,573,994, the entire contents of each of which are incorporated herein by reference.

Particular aspects of the electrochemical sensor 12 are constructed in accordance with thin film mask techniques to include elongated thin film conductors embedded or encased between layers of a selected insulative material such as polyimide film or sheet, and membranes. The one or more sensor electrodes 20 may be disposed at a tip end of the sensing portion 18 and exposed through one of the insulative layers for direct contact with patient blood or other body fluids, when the sensing portion 18 of the electrochemical sensor 12 is subcutaneously placed at an insertion site. The sensing portion 18 is joined to a connection portion 24 (FIG. 1) that terminates in conductive contact pads (not shown), which are also exposed through one of the insulative layers. In alternative aspects, other types of implantable sensors, such as chemical based, optical-based, or the like, may be used.

The connection portion 24 and the conductive contact pads may be generally adapted for a direct wired electrical connection to the sensor electronics device 100 for monitoring a user's condition in response to signals derived from the one or more sensor electrodes 20. Further description of flexible thin film sensors of this general type may be found, e.g., in U.S. Patent No. 5,391,250, the entire contents of which are incorporated herein by reference. The connection portion 24 may be conveniently connected electrically to the sensor electronics device 100 by a connector block 28, as shown in FIG. 1 and also as shown and described, e.g., in U.S. Patent No. 5,482,473, the entire contents of which is incorporated herein by reference. Thus, in accordance with aspects of the present disclosure, the subcutaneous sensor set 10 may be configured to work with either a wired or a wireless characteristic monitor system.

The one or more sensor electrodes 20 may be used in a variety of sensing applications and may be configured in a variety of ways. For example, the one or more sensor electrodes 20 may be used in physiological parameter sensing applications in which some type of biomolecule is used as a catalytic agent. For example, the one or more sensor electrodes 20 may be used in a glucose and oxygen sensor having a glucose oxidase (GOx) enzyme catalyzing a reaction with the one or more sensor electrodes 20. The reaction produces Gluconic Acid (C₆H₁₂O₇) and Hydrogen Peroxide (H₂O₂) in proportion to the amount of glucose present.

The one or more sensor electrodes 20, along with a biomolecule or some other catalytic agent, may be placed in a human body in a vascular or non-vascular environment. For example, the one or more sensor electrodes 20 and biomolecule may be placed in a vein and be subjected to a blood stream, or may be placed in a subcutaneous or peritoneal region of the human body.

With continued reference to FIG. 1, the sensor electronics device 100 includes a power source 110, a sensor interface 122, processing electronics 124, and data formatting electronics 128. The sensor electronics device 100 may be coupled to the sensor set 10 by a cable 102 through a connector portion 104 that is electrically coupled to the connector block 28 of the connection portion 24. In an alternative aspect, the cable 102 may be omitted. In this aspect of the present disclosure, the sensor electronics device 100 may include a suitable connector (not shown) for direct connection to the connector portion 104 of the sensor set 10. The sensor set 10 may be modified to have the connector portion 104 positioned at a different location, e.g., on top of the sensor set 10 to facilitate placement of the sensor electronics device 100 over the sensor set 10.

In aspects of the present disclosure, the sensor interface 122, the processing electronics 124, and the data formatting electronics 128 are formed as separate semiconductor chips, however, alternative aspects may combine the various semiconductor chips into a single or multiple customized semiconductor chips. The sensor interface 122 connects with the cable 102 that is connected with the sensor set 10.

The power source 110 may be a battery. In aspects of the present disclosure, the battery may include three series silver oxide battery cells. In alternative aspects, different battery chemistries may be utilized, such as lithium based chemistries, alkaline batteries, nickel metalhydride, or the like, and a different number of batteries may be used. The sensor electronics device 100 provides power to the sensor set 10 via the power source 110, through the cable 102 and cable connector portion 104. In an aspect of the present disclosure, the power is a voltage provided to the sensor set 10. In another aspect of the present disclosure, the power is a current provided to the sensor set 10. In yet another aspect of the present disclosure, the power is a voltage provided at a specific voltage to the sensor set 10.

FIG. 2 illustrates the sensing portion 18 of the sensor 12 according to an aspect of the present disclosure. The sensing portion 18 generally includes one or more working electrodes 204, 208, 212, one or more counter electrodes 202, 206, 210, and a reference electrode 214. In aspects of the present disclosure, the reference electrode 214 may be located at a distal portion of the sensing portion 18 of the sensor 12. The one or more counter electrodes 202, 206, 210 may operate in pairs with the one or more the working electrodes 204, 208, 212. Generally, a voltage may be supplied between the reference electrode 214 and any one or more of the working electrodes 204, 208, 212 by adjusting the voltage at any one or more of the counter electrodes 202, 206, 210. Current may then flow from any one or more of the counter electrodes 202, 206, 210 to any one or more corresponding working electrodes 204, 208, 212. Such current may be measured to ascertain the electrochemical reaction between the one or more sensor electrodes 20 and a biomolecule of a sensor that has been placed in the vicinity of the one or more sensor electrodes 20 and used as a catalyzing agent.

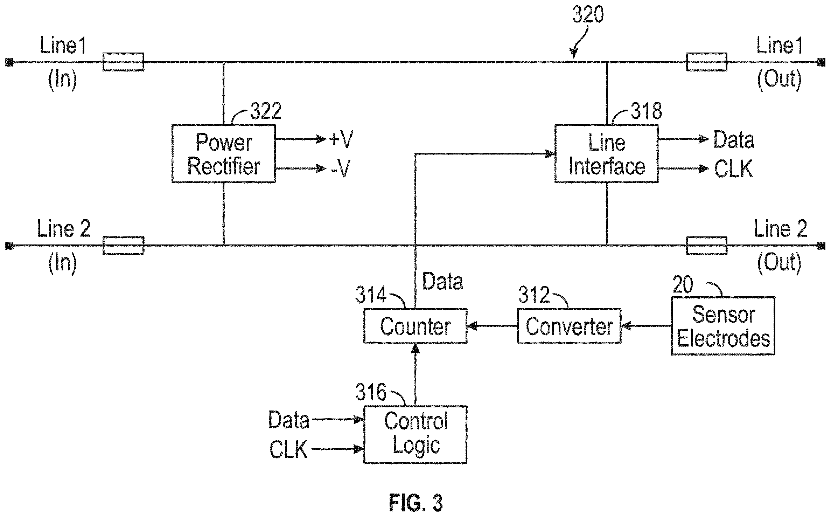

FIG. 3 illustrates a general block diagram of an electronic circuit for sensing an output of a sensor according to aspects of the present disclosure. The one or more sensor electrodes 20 may interface to a data converter 312, the output of which may interface to a counter 314. The counter 314 may be controlled by control logic 316. The output of the counter 314 may connect to a line interface 318. The line interface 318 may be connected to input and output lines 320 and may also connect to the control logic 316. The input and output lines 320 may also be connected to a power rectifier 322.

The one or more sensor electrodes 20 may be used in a variety of sensing applications and may be configured in a variety of ways. For example, the one or more sensor electrodes 20 may be used in physiological parameter sensing applications in which some type of biomolecule is used as a catalytic agent. For example, the one or more sensor electrodes 20 may be used in a glucose and oxygen sensor having a glucose oxidase (GOx) enzyme catalyzing a reaction with the one or more sensor electrodes 20. The one or more sensor electrodes 20, along with a biomolecule or some other catalytic agent, may be placed in a human body in a vascular or non-vascular environment. For example, the one or more sensor electrodes 20 and biomolecule may be placed in a vein and be subjected to a blood stream.

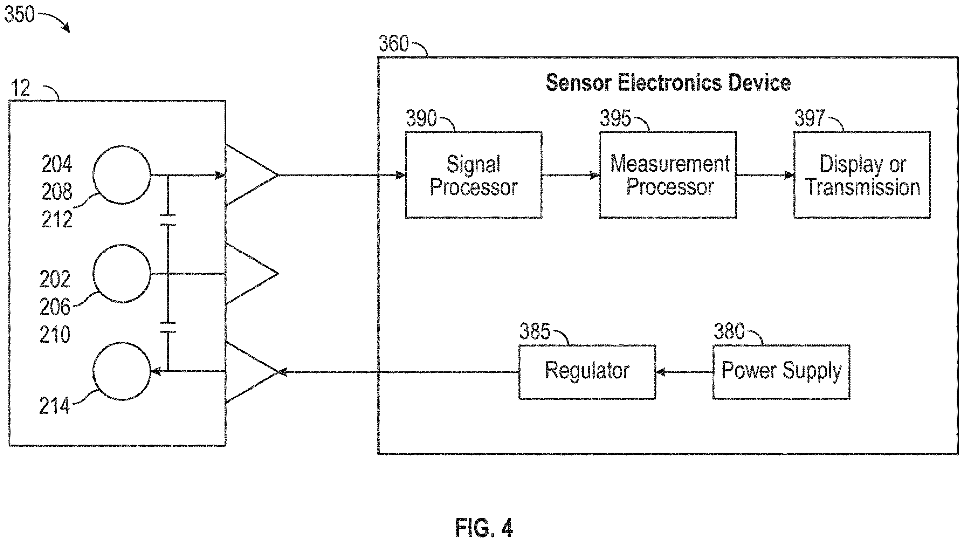

FIG. 4 illustrates a block diagram of a subcutaneous sensor set 350 including a sensor electronics device 360 and a sensor 12 including a plurality of electrodes according to an aspect of the present disclosure. The sensor 12 includes counter electrodes 202, 206, 210, a reference electrode 214, and working electrodes 204, 208, 212. The sensor electronics device 360 includes a power supply 380, a regulator 385, a signal processor 390, a measurement processor 395, and a display/transmission module 397. The power supply 380 provides power (in the form of either a voltage, a current, or a voltage including a current) to the regulator 385. The regulator 385 transmits a regulated voltage to the sensor 12. In an aspect of the present disclosure, the regulator 385 transmits a voltage to one or more of the counter electrodes 202, 206, 210 of the sensor 12.

The sensor 12 creates a sensor signal indicative of a concentration of a physiological characteristic being measured. For example, the sensor signal may be indicative of a blood glucose reading. In an aspect of the present disclosure, utilizing subcutaneous sensors, the sensor signal may represent a level of hydrogen peroxide in a subject. In an aspect of the present disclosure, where blood or cranial sensors are utilized, the amount of oxygen is being measured by the sensor 12 and is represented by the sensor signal. In an aspect of the present disclosure, utilizing implantable or long- term sensors, the sensor signal may represent a level of oxygen in the subject. The sensor signal may be measured at any one or more of the working electrodes 204, 208, 212. In an aspect of the present disclosure, the sensor signal may be a current measured at any one or more of the working electrodes 204, 208, 212. In an aspect of the present disclosure, the sensor signal may be a voltage measured at any one or more of the working electrodes 204, 208, 212.

The signal processor 390 receives the sensor signal (e.g., a measured current or voltage) after the sensor signal is measured at any one or more of the working electrodes 204, 208, 212 of the sensor 12. The signal processor 390 processes the sensor signal and generates a processed sensor signal. The measurement processor 395 receives the processed sensor signal and calibrates the processed sensor signal utilizing reference values. In an aspect of the present disclosure, the reference values are stored in a reference memory (not shown) and provided to the measurement processor 395. The measurement processor 395 generates sensor measurements. The sensor measurements may be stored in a measurement memory (not shown). The sensor measurements may be sent to the display/transmission device 397 to be either displayed on a display included on a housing of the sensor electronics device 360 or transmitted to an external device.

The sensor electronics device 360 may include a display device that serves to display physiological characteristics readings. The sensor electronics device 360 may also be installed in a desktop computer, a pager, a television including communications capabilities, a laptop computer, a server, a network computer, a personal digital assistant (PDA), a portable telephone including computer functions, an infusion pump including a display, a glucose sensor including a display, and/or a combination infusion pump/glucose sensor. The sensor electronics device 360 may be housed in a cellular phone, a smartphone, a network device, a home network device, and/or other appliance connected to a home network.

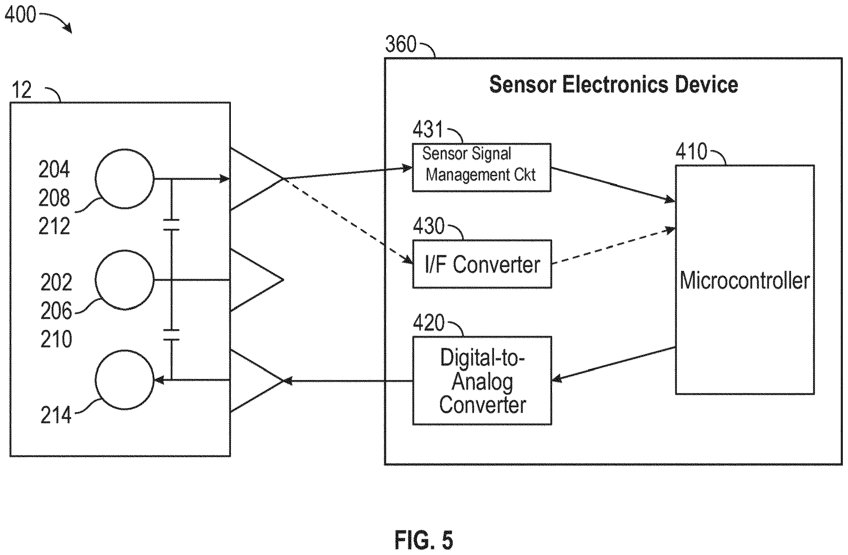

FIG. 5 illustrates an alternative aspect including a sensor and a sensor electronics device according to an aspect of the present disclosure. The sensor set or sensor system 400 includes a sensor electronics device 360 and a sensor 12. The sensor 12 includes one or more counter electrodes 202, 206, 210, a reference electrode 214, and one or more working electrodes 204, 208, 212. The sensor electronics device 360 includes a microcontroller 410 and a digital-to-analog converter (DAC) 420. The sensor electronics device 360 may also include a current-to-frequency converter (I/F converter) 430.

The microcontroller 410 includes software program code or programmable logic, which, when executed, causes the microcontroller 410 to transmit a signal to the DAC 420, where the signal is representative of a voltage level or value that is to be applied to the sensor 12. The DAC 420 receives the signal and generates the voltage value at the level instructed by the microcontroller 410. In aspects of the present disclosure, the microcontroller 410 may change the representation of the voltage level in the signal frequently or infrequently. Illustratively, the signal from the microcontroller 410 may instruct the DAC 420 to apply a first voltage value for one second and a second voltage value for two seconds.

The sensor 12 may receive the voltage level or value. In an aspect of the present disclosure, any one or more of the counter electrodes 202, 206, 210 may receive the output of an operational amplifier (not shown), which has as inputs the reference voltage and the voltage value from the DAC 420. The application of the voltage level causes the sensor 12 to create a sensor signal indicative of a concentration of a physiological characteristic being measured. In an aspect of the present disclosure, the microcontroller 410 may measure the sensor signal (e.g., a current value) from any one or more of the working electrodes 204, 208, 212. Illustratively, a sensor signal measurement circuit 431 may measure the sensor signal. In an aspect of the present disclosure, the sensor signal measurement circuit 431 may include a resistor, and the current may be passed through the resistor to measure the value of the sensor signal. In an aspect of the present disclosure, the sensor signal may be a current-level signal. The I/F converter 430 may measure the sensor signal in terms of a current reading, convert it to a frequency-based sensor signal or an EIS signal, and transmit the frequency-based sensor signal or EIS signal to the microcontroller 410. In aspects of the present disclosure, the microcontroller 410 may be able to receive frequency-based sensor signals easier than non-frequency-based sensor signals. The microcontroller 410 receives the sensor signal, whether frequency-based or non-frequency-based, and determines a value for the physiological characteristic of a subject, such as a blood glucose level. The microcontroller 410 may include program code, which when executed or run, is able to receive the sensor signal and convert the sensor signal to a physiological characteristic value.

In one aspect of the present disclosure, the microcontroller 410 may convert the sensor signal to a blood glucose level. While converting the sensor signal to a blood glucose value, the microcontroller 410 may use one or more models, which are specific ways to use the sensor signal to calculate the blood glucose value. In some aspects of the present disclosure, the microcontroller 410 may utilize measurements (e.g., sensor signals and EIS signals from the sensor 12) stored within an internal memory in order to determine the blood glucose level of the subject. In some aspects of the present disclosure, the microcontroller 410 may utilize measurements stored within a memory external to the microcontroller 410 to assist in determining the blood glucose level of the subject.

After the physiological characteristic value is determined by the microcontroller 410, the microcontroller 410 may store measurements of the physiological characteristic values for a number of time periods. For example, a blood glucose (BG) value may be sent to the microcontroller 410 from the sensor 12 every second or five seconds, and the microcontroller 410 may save sensor measurements for five minutes or ten minutes of BG value readings. The microcontroller 410 may transfer the measurements of the physiological characteristic values to a display on the sensor electronics device 360. In one aspect of the present disclosure, the microcontroller 410 may transfer the measurements of the physiological characteristic values to an output interface (not shown) of the microcontroller 410. The output interface of the microcontroller 410 may transfer the measurements of the physiological characteristic values, e.g., blood glucose values, to an external device, e.g., an infusion pump, a combined infusion pump/glucose meter, a computer, a personal digital assistant, a pager, a network appliance, a server, a cellular phone, or any computing device.

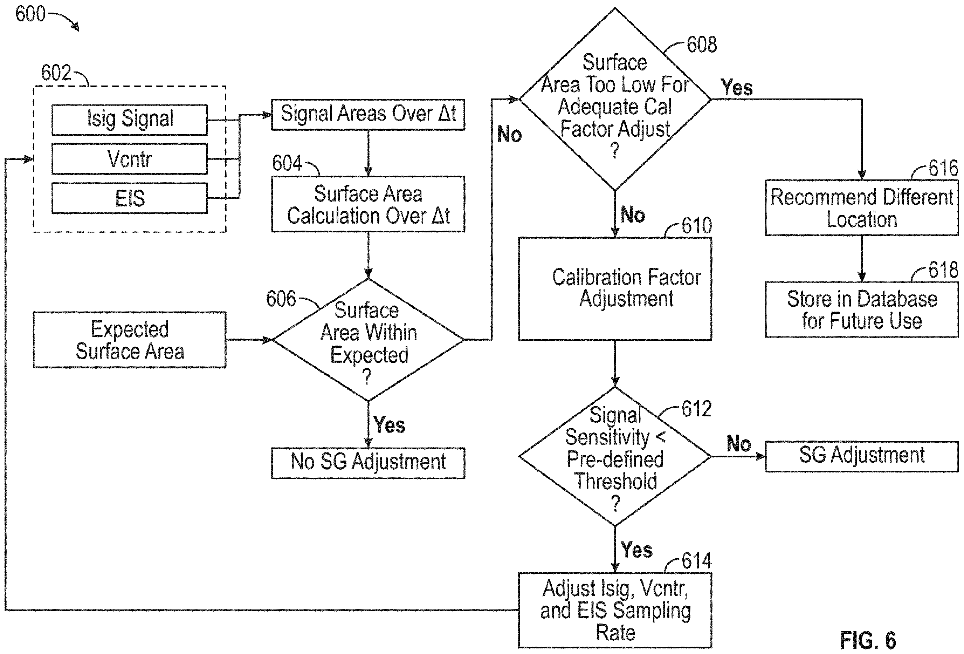

Referring to FIG. 6, there is shown a flow chart of an exemplary computer-implemented approach 600 for assessing glucose sensor reaction surface area in accordance with aspects of the present disclosure. Although the blocks of FIG. 6 are shown in a particular order, the blocks need not all be performed in the specified order, and certain blocks can be performed in another order. For simplicity, FIG. 6 will be described below, with the controller 410 performing the operations. However, in various aspects, the operations of FIG. 6 may be performed in part by the controller 410 of FIG. 5 and in part by another device, such as a remote server. These variations are contemplated to be within the scope of the present disclosure.

The approach 600 provides the benefits of using various sensor signals (in vivo and/or invitro) to determine if the glucose sensor is encapsulated, by determining a reduction in surface area vs. an expected surface area of the glucose sensor. The approach 600 is able to compensate for the reduction in available sensor surface area of the glucose sensor to ensure accurate and reliable sensor data while potentially extending the life of the glucose sensor.

Initially, at block 602, the controller 410 causes the sensor electronics to periodically measure electrode current signal (Isig) values for one or more of the working electrodes 204, 208, 212, and counter voltage (Vcntr) values for a glucose sensor. For example, the sensor electronics device 360 may periodically measure Isig in approximately one-minute intervals or in approximately five minute intervals. In aspects of the present disclosure, the controller 410 may generate EIS parameter values for one or more of the working electrodes 204, 208, 212 based on an EIS procedure. The EIS parameter values include at least one of real impedance, imaginary impedance, or magnitude of vector of real and imaginary portions of impedance. For example, the EIS parameter may include an imaginary impedance at about 0.1 Hz and/or a real impedance at about 1 KHz. In aspects of the present disclosure, the controller 410 may generate EIS parameter values in approximately 30 minute intervals.

At block 604, the controller 410 estimates the reaction surface area of the glucose sensor based on Isig values, Vcntr values, and/or the EIS parameter values. It is contemplated that any of these parameters or values may be used alone or in combination with each other for estimating reaction surface area, for example, using the combination of Isig and imaginary impedance.

In aspects of the present disclosure, the reaction surface area may be determined over a change in time. For example, the glucose sensor may have about 33 percent of the proximal surface area (e.g., the proximal working electrode 204 and the proximal counter electrode 202) covered by a biofilm. The controller 410 may estimate, based on an Isig value for this particular glucose sensor, that only about 66% of the reaction surface area is useable for sensing.

At block 606, the controller 410 compares the estimated reaction surface area to an expected reaction surface area of the glucose sensor. In the case that the estimated reaction surface area of the glucose sensor is within an expected range, then the controller 410 does not cause the glucose sensor to adjust the sensor glucose (SG) value, such that the SG value is maintained.

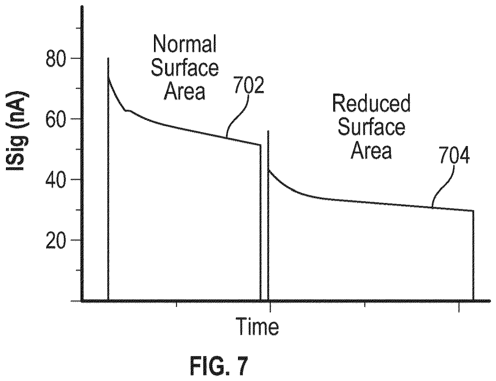

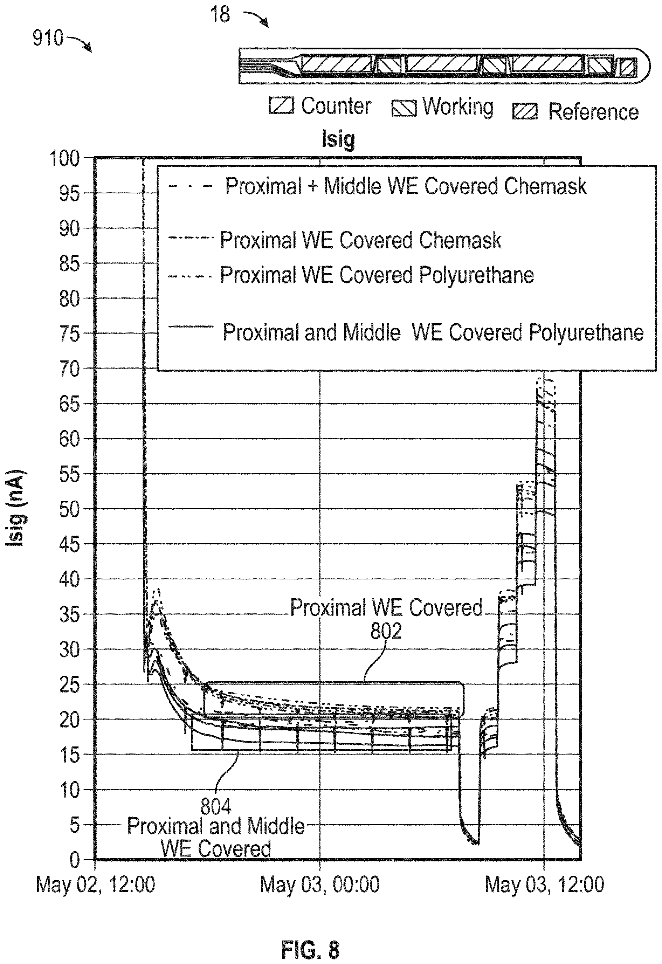

In aspects of the present disclosure, the expected reaction surface area of the glucose sensor may be based on an area under a signal curve being proportional to the reaction surface area. For example, FIG. 7 is a graph showing Isig vs. time for a glucose sensor. As illustrated by the graph of FIG. 7, a glucose sensor that has a normal amount of reaction surface area generally has a higher area under the curve 702. As the sensor reaction surface area is reduced, the area under the curve 704 is reduced. The signal curve may be based on Isig values, Vcntr values, and/or the EIS parameter values over time (FIGS. 8-10). The area under the signal curve may be determined, for example, by performing an integral between two points on the curve.

In aspects of the present disclosure, the expected reaction surface area of the glucose sensor may be based on an average value of the measured parameter (e.g., Isig, Vcntr, and/or EIS) over time. For example, a sliding window may be used to calculate an average over a predetermined time period.

Next, at block 608, the controller 410 determines if the reaction surface area is too small for calibration factor adjustment based on comparing the determined difference to a predetermined threshold value. For example, if the predetermined threshold value is about 80% and the determined difference is about 90%, then the controller 410 may determine that the reaction surface area is too small for calibration factor adjustment.

In the case that the reaction surface area is too low, the controller 410 at block 616 transmits a message recommending a different physical location for insertion of the glucose sensor. For example, the controller 410 may display on a display a message such as "please reinsert the glucose sensor at a new location." In aspects of the present disclosure, the controller 410 at block 618 may store the old and/or new physical location in a database for future use. In aspects of the present disclosure, the glucose sensor reaction surface area may be stored in a database and associated with a particular glucose sensor by a serial number of that particular glucose sensor. In aspects of the present disclosure, the controller 410 may suggest a specific different physical location for insertion of the glucose sensor based on the current physical location of the glucose sensor, in response to the reaction surface area and/or the signal sensitivity being below a threshold value.

In the case that the reaction surface area is not too small for calibration factor adjustment, the controller 410 at block 610 adjusts the calibration factor to adjust the signal sensitivity of the glucose sensor. For example, if the predetermined threshold value is about 60% and the determined difference is about 40%, then the controller 410 may determine that the reaction surface area is appropriate for calibration factor adjustment.

In aspects of the present disclosure, the calibration factor adjustment may be based on the difference between the estimated reaction surface area and the expected reaction surface area. For example, the glucose sensor may initially start with a factory calibration factor. The factory calibration factor may be adjusted or replaced with a calibration factor that is calculated based on the difference between the estimated reaction surface area and the expected reaction surface area. In aspects of the present disclosure, the controller 410 may cause the glucose sensor to perform a calibration factor adjustment when the determined difference is below the predetermined threshold. The calibration factor adjustment may be based on the difference between the estimated reaction surface area and the expected reaction surface area.

At block 612, the controller 410 may determine if the signal sensitivity is below a predetermined threshold value. If the signal sensitivity is above the predetermined threshold, then the controller 410 adjusts the SG value. In aspects of the present disclosure, the SG value adjustment may be performed in a moving window of about five to about seven minutes.

In the case that the signal sensitivity is determined to be below the predetermined threshold value, the controller 410 at block 614 adjusts a sample rate for measuring Isig, Vcntr, and/or EIS in response to the determined signal sensitivity being below the predetermined threshold value.

In aspects of the present disclosure, the controller 410 may cause an infusion pump to dispense insulin to provide treatment to a user, in response to the adjusted SG levels.

Referring now to FIGS. 8-10, graphs are shown for Isig and EIS parameters over time for sensors with either 33% or 66% reduction in reaction surface area. Two test groups of sensing portions 18 of sensors 12 were tested by having either about 33% (test group 1) or 66% (test group 2) of their electrode surface area encapsulated by different example materials (e.g., chemask or polyurethane).

FIG. 8 is a graph illustrating Isig over time for electrodes with varying amounts of reaction surface area. For Isig measurements, the traces for test group 1, referenced in FIG. 8 by reference number 802, have higher average Isig values over time as compared to the traces for test group 2, referenced in FIG. 8 by reference number 804.

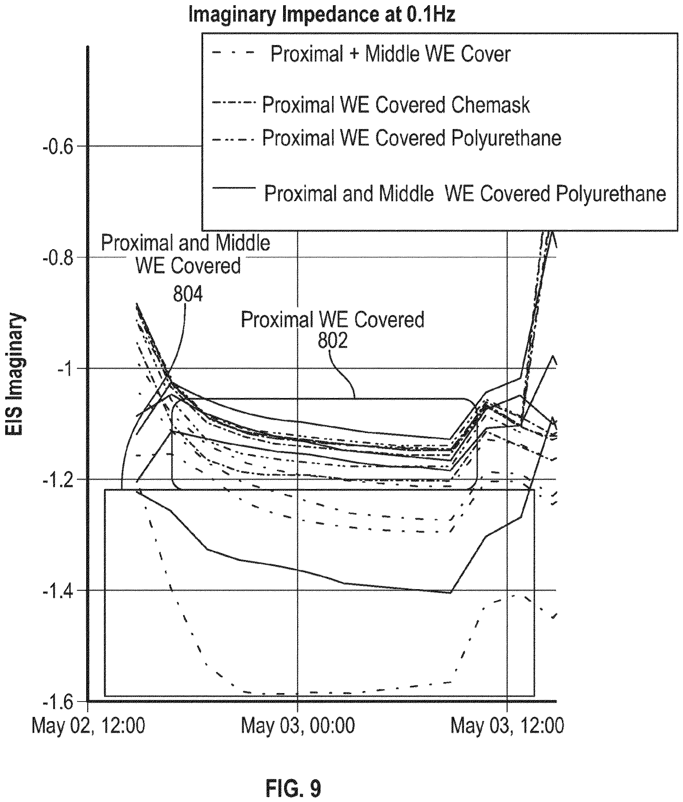

FIG. 9 is a graph illustrating imaginary impedance at 0.1 Hz over time for electrodes with varying amounts of reaction surface area. For imaginary impedance measurements at 0.1 Hz, the traces for test group 1, referenced in FIG. 9 by reference number 802, have higher average imaginary impedance values over time as compared to the traces for test group 2, referenced in FIG. 9 by reference number 804. Although imaginary impedance measurements are shown at about 0.1 Hz, other frequencies are contemplated.

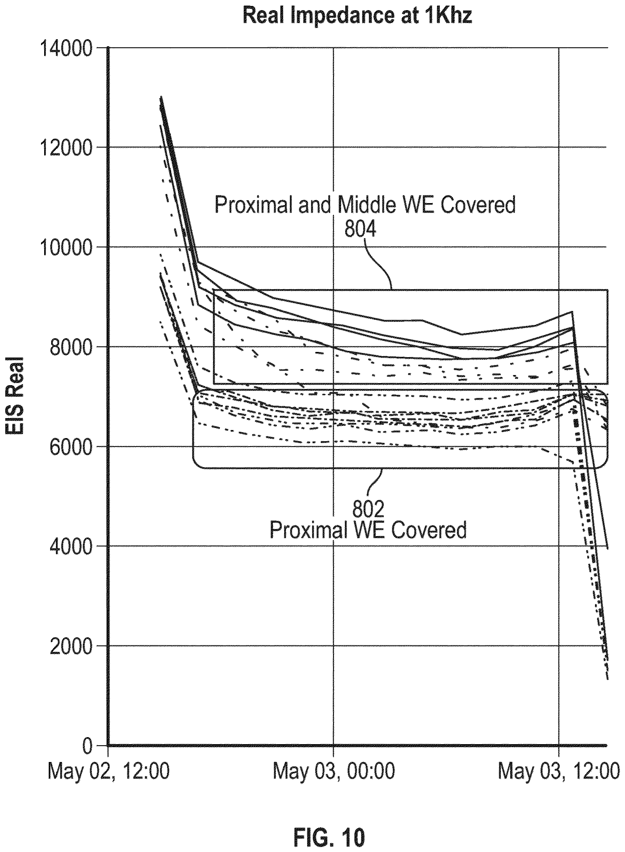

FIG. 10 is a graph illustrating real impedance at 1 KHz over time for electrodes with varying amounts of reaction surface area. For real impedance measurements at 1 KHz, the traces for test group 2, referenced in FIG. 10 by reference number 804, have higher average real impedance values over time as compared to the traces for test group 1, referenced in FIG. 10 by reference number 802. Thus, as more of the reaction surface area is obscured or covered, the larger the real impedance measurements over time. Although real impedance measurements are shown at about 1 KHz, other frequencies are contemplated.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, the above-described servers and computing devices.

While the description above refers to particular aspects of the present disclosure, it will be understood that many modifications may be made without departing from the spirit thereof. Additional blocks and changes to the order of the algorithms can be made while still performing the key teachings of the present disclosure. Thus, the accompanying claims are intended to cover such modifications as would fall within the true scope and spirit of the present disclosure. The presently disclosed aspects are, therefore, to be considered in all respects as illustrative and not restrictive, the scope of the disclosure being indicated by the appended claims rather than the foregoing description. Unless the context indicates otherwise, any aspect disclosed herein may be combined with any other aspect or aspects disclosed herein. All changes that come within the meaning of, and range of, equivalency of the claims are intended to be embraced therein.

Further disclosed herein is the subject-matter of the following clauses:
1. A glucose sensor, comprising:
   a working electrode;
   sensor electronics configured to periodically measure electrode current signal (Isig) values for the working electrode and counter voltage (Vcntr) values for the glucose sensor;
   one or more processors; and
   one or more processor-readable media storing instructions which, when executed by the one or more processors, cause performance of:
      generating Electrochemical Impedance Spectroscopy (EIS) parameter values for the working electrode based on an EIS procedure;
      estimating a reaction surface area of the glucose sensor based on least one of Isig values, Vcntr values, or the EIS parameter values;
      determining a difference between the estimated reaction surface area and an expected reaction surface area of the glucose sensor;
      comparing the determined difference to a predetermined threshold value; and
      adjusting a calibration factor when the determined difference is below the predetermined threshold value.
2. The glucose sensor of clause 1, wherein the calibration factor adjustment is based on the difference between the estimated reaction surface area and the expected reaction surface area.
3. The glucose sensor of clause 1 or 2, wherein the one or more processor-readable media further store instructions which, when executed by the one or more processors, cause performance of:
   determining if a signal sensitivity is below a predetermined threshold value.
4. The glucose sensor of clause 3 or of any of the clauses 1 to 3, wherein the one or more processor-readable media further store instructions which, when executed by the one or more processors, cause performance of: adjusting a sample rate for measuring at least one of Isig, Vcntr, or EIS in response to the signal sensitivity being below the predetermined threshold value.
5. The glucose sensor of clause 1 or of any of the clauses 1 to 4, wherein the EIS parameter values include at least one of real impedance, an imaginary impedance, or a magnitude of vector of real and imaginary portions of impedance.
6. The glucose sensor of clause 1 or of any of the clauses 1 to 5, wherein the EIS parameter is an imaginary impedance at 0.1 Hz or a real impedance at 1 KHz.
7. The glucose sensor of clause 1 or of any of the clauses 1 to 6, wherein estimating the reaction surface area of the glucose sensor includes estimating the reaction surface area over time.
8. The glucose sensor of clause 1 or of any of the clauses 1 to 7, wherein the one or more processor-readable media further store instructions which, when executed by the one or more processors, cause performance of: providing an indication for a user to move the glucose sensor to a different physical wear location.
9. The glucose sensor of clause 1 or of any of the clauses 1 to 8, wherein the expected reaction surface area of the glucose sensor is based on an area under a signal curve being proportional to the reaction surface area.
10. The glucose sensor of clause 9 or of any of the clauses 1 to 9, wherein the signal curve is based on least one of Isig values, Vcntr values, or the EIS parameter values over time.
11. One or more non-transitory processor-readable media storing instructions which, when executed by one or more processors, cause performance of:
   periodically measuring electrode current signal (Isig) values for a working electrode and counter voltage (Vcntr) values for a glucose sensor;
   generating Electrochemical Impedance Spectroscopy (EIS) parameter values for the working electrode based on an EIS procedure;
   estimating a reaction surface area of the glucose sensor based on least one of Isig values, Vcntr values, or the EIS parameter values;
   determining a difference between the estimated reaction surface area and an expected reaction surface area of the glucose sensor;
   comparing the determined difference to a predetermined threshold value; and
   adjusting a calibration factor when the determined difference is below the predetermined threshold value.
12. The one or more non-transitory processor-readable media of clause 11, wherein the one or more non-transitory processor-readable media store further instructions which, when executed by the one or more processors, cause performance of: wherein the calibration factor adjustment is based on the difference between the estimated reaction surface area and the expected reaction surface area.
13. The one or more non-transitory processor-readable media of clause 11 or 12, wherein the one or more non-transitory processor-readable media store further instructions which, when executed by the one or more processors, cause performance of:
   determining if a signal sensitivity is below a predetermined threshold value; and
   adjusting a sample rate for measuring at least one of Isig, Vcntr, or EIS in response to the signal sensitivity being below the predetermined threshold value.
14. The one or more non-transitory processor-readable media of clause 11 or of any of the clauses 11 to 13, wherein the EIS parameter values include at least one of a real impedance, an imaginary impedance, or a magnitude of vector of real and imaginary portions of impedance.
15. The one or more non-transitory processor-readable media of clause 11 or of any of the clauses 11 to 14, wherein the EIS parameter is a real impedance at 1 KHz.
16. The one or more non-transitory processor-readable media of clause 11 or of any of the clauses 11 to 15, wherein the EIS parameter is an imaginary impedance at 0.1 Hz.
17. The one or more non-transitory processor-readable media of clause 11 or of any of the clauses 11 to 16, wherein estimating the reaction surface area of the glucose sensor includes estimating the reaction surface area over time.
18. The one or more non-transitory processor-readable media of clauses 11 or of any of the clauses 11 to 17, wherein the one or more non-transitory processor-readable media store further instructions which, when executed by the one or more processors, cause performance of: providing an indication for a user to move the glucose sensor to a different physical wear location.
19. The one or more non-transitory processor-readable media of clause 11 or of any of the clauses 11 to 18, wherein the expected reaction surface area of the glucose sensor is based on an area under a signal curve being proportional to the reaction surface area, and wherein the signal curve is based on least one of Isig values, Vcntr values, or the EIS parameter values over time.
20. A system, comprising:
   one or more processors; and
   one or more processor-readable media storing instructions which, when executed by the one or more processors, cause performance of:
      periodically measuring electrode current signal (Isig) values for a working electrode and counter voltage (Vcntr) values for a glucose sensor;
      generating Electrochemical Impedance Spectroscopy (EIS) parameter values for the working electrode based on an EIS procedure;
      estimating a reaction surface area of the glucose sensor based on least one of Isig values, Vcntr values, or the EIS parameter values;
      determining a difference between the estimated reaction surface area and an expected reaction surface area of the glucose sensor;
      comparing the determined difference to a predetermined threshold value; and
      adjusting a calibration factor when the determined difference is below the predetermined threshold value.

## Claims

1. A glucose sensor, comprising:
a working electrode;
sensor electronics configured to periodically measure electrode current signal (Isig) values for the working electrode and counter voltage (Vcntr) values for the glucose sensor;
one or more processors; and
one or more processor-readable media storing instructions which, when executed by the one or more processors, cause performance of:
generating Electrochemical Impedance Spectroscopy (EIS) parameter values for the working electrode based on an EIS procedure;
estimating a reaction surface area of the glucose sensor based on least one of Isig values, Vcntr values, or the EIS parameter values;
determining a difference between the estimated reaction surface area and an expected reaction surface area of the glucose sensor;
comparing the determined difference to a predetermined threshold value; and
adjusting a calibration factor when the determined difference is below the predetermined threshold value.

2. The glucose sensor of claim 1, wherein the calibration factor adjustment is based on the difference between the estimated reaction surface area and the expected reaction surface area.

3. The glucose sensor of claim 1 or 2, wherein the one or more processor-readable media further store instructions which, when executed by the one or more processors, cause performance of:
determining if a signal sensitivity is below a predetermined threshold value,
particularly
wherein the one or more processor-readable media further store instructions which, when executed by the one or more processors, cause performance of: adjusting a sample rate for measuring at least one of Isig, Vcntr, or EIS in response to the signal sensitivity being below the predetermined threshold value.

4. The glucose sensor of any of the claims 1 to 3, wherein the EIS parameter values include at least one of real impedance, an imaginary impedance, or a magnitude of vector of real and imaginary portions of impedance, and/or
wherein the EIS parameter is an imaginary impedance at 0.1 Hz or a real impedance at 1 KHz.

5. The glucose sensor of any of the claims 1 to 4, wherein estimating the reaction surface area of the glucose sensor includes estimating the reaction surface area over time.

6. The glucose sensor of any of the claims 1 to 5, wherein the one or more processor-readable media further store instructions which, when executed by the one or more processors, cause performance of: providing an indication for a user to move the glucose sensor to a different physical wear location.

7. The glucose sensor of any of the claims 1 to 6, wherein the expected reaction surface area of the glucose sensor is based on an area under a signal curve being proportional to the reaction surface area,
particularly
wherein the signal curve is based on least one of Isig values, Vcntr values, or the EIS parameter values over time.

8. One or more non-transitory processor-readable media storing instructions which, when executed by one or more processors, cause performance of:
periodically measuring electrode current signal (Isig) values for a working electrode and counter voltage (Vcntr) values for a glucose sensor;
generating Electrochemical Impedance Spectroscopy (EIS) parameter values for the working electrode based on an EIS procedure;
estimating a reaction surface area of the glucose sensor based on least one of Isig values, Vcntr values, or the EIS parameter values;
determining a difference between the estimated reaction surface area and an expected reaction surface area of the glucose sensor;
comparing the determined difference to a predetermined threshold value; and
adjusting a calibration factor when the determined difference is below the predetermined threshold value.

9. The one or more non-transitory processor-readable media of claim 8, wherein the one or more non-transitory processor-readable media store further instructions which, when executed by the one or more processors, cause performance of: wherein the calibration factor adjustment is based on the difference between the estimated reaction surface area and the expected reaction surface area.

10. The one or more non-transitory processor-readable media of claim 8 or 9, wherein the one or more non-transitory processor-readable media store further instructions which, when executed by the one or more processors, cause performance of:
determining if a signal sensitivity is below a predetermined threshold value; and
adjusting a sample rate for measuring at least one of Isig, Vcntr, or EIS in response to the signal sensitivity being below the predetermined threshold value.

11. The one or more non-transitory processor-readable media of any of the claims 8 to 10,
wherein the EIS parameter values include at least one of a real impedance, an imaginary impedance, or a magnitude of vector of real and imaginary portions of impedance, and/or
wherein the EIS parameter is a real impedance at 1 KHz, and/or
wherein the EIS parameter is an imaginary impedance at 0.1 Hz.

12. The one or more non-transitory processor-readable media of any of the claims 8 to 11, wherein estimating the reaction surface area of the glucose sensor includes estimating the reaction surface area over time.

13. The one or more non-transitory processor-readable media of any of the claims 8 to 12, wherein the one or more non-transitory processor-readable media store further instructions which, when executed by the one or more processors, cause performance of: providing an indication for a user to move the glucose sensor to a different physical wear location.

14. The one or more non-transitory processor-readable media of any of the claims 8 to 13, wherein the expected reaction surface area of the glucose sensor is based on an area under a signal curve being proportional to the reaction surface area, and wherein the signal curve is based on least one of Isig values, Vcntr values, or the EIS parameter values over time.

15. A system, comprising:
one or more processors; and
one or more processor-readable media storing instructions which, when executed by the one or more processors, cause performance of:
periodically measuring electrode current signal (Isig) values for a working electrode and counter voltage (Vcntr) values for a glucose sensor;
generating Electrochemical Impedance Spectroscopy (EIS) parameter values for the working electrode based on an EIS procedure;
estimating a reaction surface area of the glucose sensor based on least one of Isig values, Vcntr values, or the EIS parameter values;
determining a difference between the estimated reaction surface area and an expected reaction surface area of the glucose sensor;
comparing the determined difference to a predetermined threshold value; and
adjusting a calibration factor when the determined difference is below the predetermined threshold value.
